# EUROPEAN PATENT SPECIFICATION

(11) **EP 3 678 503 B1**
(45) Date of publication and mention of the grant of the patent: **27.12.2023**
(21) Application number: 18769809.7
(22) Date of filing: 04.09.2018
(51) Int. Cl.: A24F 40/50, A24F 40/60, A24F 40/10

(54) **MEMS SOUND GENERATION FOR AEROSOL-GENERATING DEVICES AND RELATED USER INTERFACES AND METHODS**
MEMS-TONERZEUGUNG FÜR AEROSOLERZEUGUNGSVORRICHTUNGEN UND ZUGEHÖRIGES VERFAHREN
GÉNÉRATION DE SONS MEMS POUR DES DISPOSITIFS DE GÉNÉRATION D'AÉROSOL ET PROCÉDÉ ASSOCIÉ

(30) Priority: 07.09.2017 EP 17189834
(43) Date of publication of application: 15.07.2020
(73) Proprietor: Philip Morris Products S.A., 2000 Neuchâtel (CH)
(72) Inventor: LAUENSTEIN, Stefan, 2000 Neuchâtel (CH); BESSANT, Michel, 2000 Neuchâtel (CH)
(74) Representative: Kelvey, Adam Charles
(86) International application number: PCT/IB2018/056750
(87) International publication number: WO 2019/049028

(56) References cited:
- WO-A1-2016/023809
- KR-A- 20020 067 473
- US-A1- 2014 246 035
- US-A1- 2015 181 930
- US-A1- 2016 128 389
- US-A1- 2016 219 933
- US-A1- 2016 360 320
- US-A1- 2017 020 196

## Description

This disclosure relates to sound generation. In particular, this disclosure relates to MEMS sound generation for an aerosol-generating device and related user interfaces and methods.

Handheld aerosol-generating devices, such as cartomizer electronic cigarettes, are known, which utilize liquid to be evaporated or tobacco material to be heated to generate an inhalable aerosol. These devices may provide an alternative experience to conventional combustion cigarettes. Some devices may adopt a similar look and feel to conventional cigarettes, which may be familiar, easy to handle, portable, and easy to manufacture. Some devices have an internal breath-activated switch or button switch to activate the generation, or release, of the inhalable aerosol.

US 2017/020196 A1 discloses an apparatus comprising a vaporizer component configured for vaporizing the vaporizable material to generate a vapor, an audio output device, configured for outputting one or more of the plurality of audio files, and a processor, configured for receiving the status of the vaporizer component, determining a first audio file of a plurality of audio files based on the status, determining a first characteristic based on the status, and causing the audio output device to output the first audio file according to the first characteristic.

Whereas some smoking materials have characteristic sounds during use, many electronic cigarettes do not produce sound. Some electronic cigarettes that do produce sound do not adequately reproduce the experience of using smoking material in a convincing manner, particularly if the sound is complex and intricate, such as the sound of burning kretek in Indonesian cigarettes, which may be described as a "crackling" sound. This inadequate experience may be undesirable to users of such conventional cigarettes. Further, electronic cigarettes may inherently produce sounds during use that may be undesirable to users. Furthermore, electronic cigarettes often operate differently than conventional cigarettes and may have features that are unfamiliar to users.

It would be desirable to provide users with an improved experience with aerosol-generating devices that adequately emulates the sound of conventional uses of smoking material in a convincing manner. It would also be desirable to provide users with the option of mitigating undesirable sounds from aerosol-generating devices. It would further be desirable to help users understand how to use, configure, and maintain the aerosol-generating devices.

This disclosure provides a MEMS sound generator used to produce sound for an aerosol-generating device. The MEMS sound generator may compress air to produce sound. The MEMS sound generator may provide sound using a plurality of drivers to produce sound at a plurality of frequencies. The sound may be described as "high fidelity." The sound may emulate the sound of one or more conventional uses of smoking materials, emulate a masking noise, or include information. The sound generated may be modulated based on a user's puff. A user interface may be provided to configure the aerosol-generating device.

In one or more aspects, an aerosol-generating device includes an aerosolizer to generate aerosol from an aerosol-generating substrate. The aerosol-generating device also includes a MEMS sound generator. The aerosol-generating device further includes a controller operatively coupled to initiate the MEMS sound generator into producing sound.

In one or more aspects, a method for use with an aerosol-generating device includes detecting an action of a user using the aerosol-generating device. The method also includes producing sound using a MEMS sound generator in response to the action of the user.

The present invention is defined by an aerosol-generating device according to claim 1, a method for use with an aerosol-generating device according to claim 9, a non-transitory computer readable storage medium according to claim 12, and a system according to claim 13.

In one or more aspects, the MEMS sound generator may compress air to produce the sound.

In one or more aspects, the MEMS sound generator may include a plurality of pressure-generating drivers arranged into an array to produce a plurality of different frequencies.

In one or more aspects, the sound may emulate the sound of a conventional use of a smoking material.

In one or more aspects, the sound may emulate the sound of burning kretek.

In one or more aspects, the sound may emulate a masking noise.

In one or more aspects, the sound may include information for a user.

In one or more aspects, an actuator may be operatively coupled to the controller, and the MEMS sound generator may produce the sound information in response to engagement of the actuator.

In one or more aspects, the information includes a voice message.

In one or more aspects, the information may provide an instruction to use or maintain the aerosol-generating device, a status of the aerosol-generating device, or both.

In one or more aspects, the MEMS sound generator may produce the sound in response to a user puff.

In one or more aspects, when the sound may be modulated, in one or more of intensity and frequency, in response to the user puff.

In one or more aspects, a puff sensor may be operatively coupled to the controller to measure the user puff.

In one or more aspects, the controller and the puff sensor may be further configured to measure a characteristic of the aerosolizer to measure the user puff.

In one or more aspects, the aerosol-generating device may include the aerosol-generating substrate, and the substrate may include a nicotine material.

In one or more aspects, a user interface device may include a communication interface to communicate with the aerosol-generating device. The user interface device may also include a display having a user interface to present one or more graphical elements to configure the aerosol-generating device. The user interface device may further include a controller operably coupled to the display and communication interface. The controller may be configured to display the one or more graphical elements on the display. The controller may also be configured to allow a user selection using the one or more graphical elements via the user interface to configure the aerosol-generating device. The controller may further be configured to communicate with the aerosol-generating device using the communication interface to configure the aerosol-generating device based on the user selection.

In one or more aspects, the aerosol-generating device may be configured based on a configuration defining one or more of: a type of aerosol-generating substrate, a specific puff sound mode, a generic puff sound mode, an instruction mode, an error message mode, an operational message mode, a substrate detection mode, a substrate selection mode, a data download mode, a configuration mode, a volume level, and an audio quality level.

In one or more aspects, a non-transitory computer readable storage medium including a computer program stored which, when run on programmable electric circuitry, may cause the programmable electric circuitry to execute the method.

Utilizing the aerosol-generating device enables an immersive experience for the user that includes sound. Sound produced by the MEMS sound generator may be similar to, or even indistinguishable to an ordinary person from, the sound of conventional use of smoking material. For example, similar to a conventional use, when the user puffs harder (for example, draws air more quickly), a characteristic of the sound may also change. Sound produced may also mask undesirable sounds during operation of the aerosol-generating device. The user may also have access to instructions or status messages to facilitate ease of use of the aerosol-generating device.

The term "aerosol-generating device" refers to a device configured to couple to, or include, an aerosol generating substrate to generate aerosol. Preferably, the aerosol-generating device also includes an aerosolizer, such as an atomizer or heater.

The term "aerosol-generating substrate" refers to a device or substrate that releases, upon heating, volatile compounds that may form an aerosol to be inhaled by a user. Suitable aerosol-generating substrates may include plant-based material. For example, the aerosol-generating substrate may include tobacco or a tobacco-containing material containing volatile tobacco flavor compounds, which may be released from the aerosol-generating substrate upon heating. In addition, or alternatively, an aerosol-generating substrate may include a non-tobacco containing material. The aerosol-generating substrate may include homogenized plant-based material. The aerosol-generating substrate may include at least one aerosol former. The aerosol-generating substrate may include other additives and ingredients such as flavorants. Preferably, the aerosol-generating substrate is a liquid at room temperature. For example, the aerosol-generating substrate may be a liquid solution, suspension, dispersion or the like. In some preferred embodiments, the aerosol generating substrate includes glycerol, propylene glycol, water, nicotine and, optionally, one or more flavorants. Preferably, the aerosol-generating substrate includes nicotine material.

The term "tobacco" refers to a substance including tobacco, which includes tobacco blends or flavored tobacco, for example.

The term "kretek" refers to a blend of tobacco, cloves, and other optional flavors. When burned, the blend produces a characteristic crackling sound. Kretek may also refer to the crackling sound of burning cloves.

This disclosure relates to a microelectromechanical system (MEMS) sound generator for an aerosol-generating device. Although reference is made herein to aerosol-generating devices, such as electronic cigarettes, the MEMS sound generator may be used on any portable device. Various other applications will become apparent to one of skill in the art having the benefit of the present disclosure.

Any suitable MEMS sound generator can be used to produce sound. The sound generator may produce sound that covers at least the human audible range. The sound may cover at least a frequency range up to about 20 kHz, or at least between about 20 Hz and about 20 kHz. The sound may be formed of a plurality of frequencies. The decibel level capable of being produced at each of the frequencies may be sufficient to be heard by a user of the sound generator. The decibel levels may be adjustable.

The sound produced may be simple or high fidelity. An example of a simple sound may be the sound of a buzzer. Examples of high-fidelity sound include a crackling sound, a voice, or white noise. Preferably, the sound generator can produce high-fidelity sound.

The sound generator may produce sound by vibrating a membrane or compressing air to produce the sound. Preferably, the sound generator compresses air to produce sound.

The sound generator of the present invention includes a plurality of pressure-generating drivers, which may also be described as microspeakers. The pressure-generating drivers are arranged into an array. The plurality of pressure-generating drivers may produce the plurality of frequencies. Each of the drivers may produce one or more of the plurality of frequencies. Preferably, each driver produces one of the frequencies. The sound generator uses digital sound reconstruction (DSR). With DSR, the sound may be produced by the sound generator as a summation of discrete pulses produced from the array of pressure-generating drivers. Utilizing DSR may produce more accurate reproduction and less distortion than a conventional analog speaker, which varies the timing of the motion of a membrane.

The array may be disposed on one or more silicon chip or integrated circuit. Preferably, the drivers are disposed on a single silicon chip or integrated circuit. The sound generator may be described as an on-chip microspeaker array.

The sound generator may be a digital speaker. The sound generator may respond to digital signals. Digital sound data may be stored in memory and provided as one or more digital signals to the sound generator.

The sound generator may be small enough to fit into a body of an aerosol-generating device. The sound generator may fit into a volume less than or equal to about 10 mm x about 10 mm x about 10 mm. In some preferred embodiments, the sound generator may fit into a volume less than or equal to about 5 mm x about 5 mm x about 5 mm. In further preferred embodiments, the sound generator may fit into a volume of less than or equal to about 5 mm x about 5 mm x about 1.5 mm.

The sound produced by the sound generator may include a variety of sounds suitable for an aerosol-generating device. The sound may emulate another sound. The emulating sound may be produced in association with the aerosolizer being activated to generate aerosol. The sound production and aerosolization may be concurrent or at least overlapping in duration. Non-limiting examples of sounds to emulate include the sound of a conventional use of smoking material and a masking noise (for example, white noise). The sound may be initiated, produced, or triggered, in response to a user action, such as a user puff or engagement of an actuator.

When used with an aerosol-generating device, the sound generator may emulate the sound of a conventional use of a smoking material. The smoking material emulated may be kretek. The generated sound may emulate the sound of burning kretek.

The sound generator may produce or emulate a masking noise. The masking noise may be a "color" of noise, such as "white" noise. The aerosol-generating device may produce a sound, such as the sound when electronic cigarette liquid evaporates, which may be undesirable to the user or others around the user. By emulating a masking noise, such as white noise, the user or others nearby may perceive a cancellation of the undesirable sound.

White noise may be an equal, or substantially equal, intensity at all frequencies, at least in the human audible range. White noise may be emulated in practice by generating sound at random frequencies having an equal average intensity. Preferably, the audible sounds generated cover at least about 2 kHz to about 16 kHz or the entire human audible range.

In one or more embodiments, the aerosol-generating device may allow the user to select whether a specific puff sound or a generic puff sound is generated in response to a user puff.

Additionally, or alternatively, the sound may include information for a user. The sound information may be provided in the form of a voice message. Non-limiting examples of sound information provided include an instruction for the aerosol-generating device (for example, an audio tutorial or manual for using or maintaining the device) and a status of the aerosol-generating device. The status of the aerosol-generating device may include one or more of an error message, an indication of puffs left, an indication of a user interaction with the device (for example, actuator engaged), and an indication of a function of the device (for example, aerosolizer activated).

In one or more embodiments, the aerosol-generating device may allow the user to select whether a specific puff sound mode or information for the user is generated in response to a user action, such as a user puff.

In one or more embodiments, the aerosol-generating device may allow the user to generate a puff sound in response to a user puff and to generate information for the user in response to a different user action.

In one or more embodiments, the aerosol-generating device may allow the user to select whether a generic puff sound or information for a user is generated in response to a user action.

In one or more embodiments, the aerosol-generating device may allow the user to select whether a specific puff sound or voice message for a user is generated in response to a user action.

In one or more embodiments, the aerosol-generating device may allow the user to select whether a generic puff sound or voice message for a user is generated in response to a user action.

The sound generator may be used with any suitable aerosol-generating device. The sound generator may be disposed within, or at least partially within, a body of the aerosol-generating device. The body of the aerosol-generating device may include one or more passages to allow sound from the sound generator to pass to an exterior of the aerosol-generating device.

The aerosol-generating device may be a smoking material heating device. In addition to the MEMS sound generator, the aerosol-generating device may include one or more of a housing, a controller, an aerosolizer, a substrate sensor, an actuator, a battery, a puff sensor, an aerosol-generating substrate receivable in a cavity defined by the housing, and a thermal break element disposed between the cavity and the controller.

The aerosol-generating device may include an aerosolizer to generate aerosol from an aerosol-generating substrate. The controller may be operatively coupled to the aerosolizer to deliver power for aerosolizing the aerosol-generating substrate from a power source, such as a battery. The aerosol-generating substrate may be removably coupled to the aerosolizer or housing of the aerosol-generating device. The aerosol-generating substrate may be at least partially inserted, received, or disposed in the housing of the aerosol-generating device.

In some embodiments, the aerosolizer may be a heating blade that heats a smoking material substrate to generate aerosol from the smoking material. The aerosol-generating substrate may be contained in a substrate housing. The substrate may be described as, or as a content of, a heat stick. The aerosolizer may be coupled to the consumable device to aerosolize the heat stick or the heat stick contents. In some embodiments, the heating blade may be inserted into the heat stick to heat the aerosol-generating substrate. The heat provided by the heating blade to the heat stick may not burn the smoking material. The smoking material may include tobacco.

In some embodiments, the aerosolizer may include a heater, a heater coil, a chemical heat source such as a carbon heat source, or any suitable means that heats a liquid substrate to generate aerosol from a liquid substrate. The aerosolizer may receive electrical energy or power to release or generate aerosol from the liquid substrate. In some embodiments, the aerosolizer may be a heater that varies in temperature depending on the electrical energy received. For example, the heater may rise in temperature in response to a higher voltage received. The aerosolizer may be disposed adjacent to the aerosol-generating substrate. For example, the aerosolizer may be coupled adjacent to the liquid substrate.

In some embodiments, the aerosolizer may be compatible for use with an aerosol-generating substrate having a nicotine source and a lactic acid source. The nicotine source may include a sorption element, such as a PTFE wick with nicotine adsorbed thereon, which may be inserted into a chamber forming a first compartment. The lactic acid source may include a sorption element, such as a PTFE wick, with lactic acid adsorbed thereon, which may be inserted into a chamber forming a second compartment. The aerosolizer may include a heater to heat both the nicotine source and the lactic acid source. Then, the nicotine vapor may react with the lactic acid vapor in the gas phase to form an aerosol.

In some embodiments, the aerosolizer may be compatible for use with an aerosol-generating substrate having a capsule that contains nicotine particles and disposed in a cavity. During a user's inhalation, the air flow may rotate the capsule. The rotation may suspend and aerosolize the nicotine particles.

The actuator may be operatively coupled to the controller. The actuator may include a button or other type of switch. The engagement of the actuator may initiate various functionality of the aerosol-generating substrate. In some embodiments, the sound generator produces the sound information for a user in response to engagement of the actuator. In some embodiments, the aerosolizer may be activated in response to engagement of the actuator.

The puff sensor may be operatively coupled to the controller. The puff sensor may be positioned within an airflow channel in the aerosol-generating device to detect when a user inhales, or puffs, on the device. The puff may be detected by the controller using the puff sensor. Non-limiting types of puff sensors may include one or more of a vibrating membrane, a piezoelectric sensor, a mesh-like membrane, a pressure sensor (for example, a capacitive pressure sensor), and an airflow switch.

One or more of the controllers described herein may include a processor, such as a central processing unit (CPU), computer, logic array, or other device capable of directing data coming into or out of the aerosol-generating device. In some embodiments, the controller includes one or more computing devices having memory, processing, and communication hardware. The functions of the controller may be performed by hardware or as computer instructions on a non-transient computer readable storage medium.

The processor of the controller may include any one or more of a microprocessor, a controller, a microcontroller, a digital signal processor (DSP), an application specific integrated circuit (ASIC), a field-programmable gate array (FPGA), or equivalent discrete or integrated logic circuitry. In some examples, the processor may include multiple components, such as any combination of one or more microprocessors, one or more controllers, one or more DSPs, one or more ASICs, or one or more FPGAs, as well as other discrete or integrated logic circuitry. The functions attributed to the controller or processor herein may be embodied as software, firmware, hardware, or any combination thereof. While described herein as a processor-based system, an alternative controller could utilize other components such as relays and timers to achieve the desired results, either alone or in combination with a microprocessor-based system.

In one or more embodiments, the exemplary systems, methods, and interfaces may be implemented using one or more computer programs using a computing apparatus, which may include one or more processors or memory. Program code or logic described herein may be applied to input data/information to perform functionality described herein and generate desired output data/information. The output data/information may be applied as an input to one or more other devices or methods as described herein or as would be applied in a known fashion. In view of the above, it will be readily apparent that the controller functionality as described herein may be implemented in any manner known to one skilled in the art.

The controller may be operatively coupled to the sound generator. The controller may be configured to initiate the sound generator into producing a sound. The controller may include one or more of a processor, a memory, a communication interface, and a circuit board. The circuit board may be coupled to one or more of the processor, the memory, and the communication interface. The processor may be operatively coupled to the memory, the sound generator, the communication interface, the aerosolizer, and the substrate sensor.

The controller may execute a sub-routine to collect an address in memory of a puff sound. A sub-routine may fetch sound puff data at the address. A sub-routine may send puff sound data to the sound generator for producing the sound.

The memory may store one or more sounds at one or more addresses as sound data and may store other data. The memory may store a plurality of sounds. The sound data may be digital data used to produce high-fidelity sound with the sound generator. The sound data may be stored to provide sufficient frequency and amplitude content to produce a high-fidelity sound. Preferably, when uncompressed, the frequency content may contain at least about 40,000 points per second of sound. The amplitude content may be represented by multiple bits per point. For example, the amplitude content may contain about 16 bits per point. The raw digitization of high-fidelity sound may have a sampling rate of about 80 kilobytes per second (KB/s) for uncompressed sound data.

In another manner of characterization, high-fidelity sound may be described as sound containing all frequencies between 20 Hz to 20 kHz represented as 16-bit samples at a 44.1 kHz sampling rate. This high-fidelity sound may be represented by 88.2 KB/s of uncompressed sound data or 264.2 kilobytes for a 3 second puff sound.

In some embodiments, MP3 compression or any other suitable compression technique may be used to reduce the size of the sound data stored in memory compared to uncompressed sound. MP3 compression may reduce high-fidelity sound data by about 90%, or one tenth of the original sampling rate. For example, uncompressed sound data at a sampling rate of about 80 KB/s may correspond to a rate as low as about 8 KB/s when converted to compressed sound data. Any suitable compression quality may be used that still produces high-quality sound.

The memory may have enough space to store multiple puff sounds, which may be described as a library of sounds. Preferably, the memory stores two or more sounds emulating the sound of a conventional use of a smoking material. Each puff sound may have a duration ranging between about 1 second to about 5 seconds. In some embodiments, the puff sound is about 1, about 2, about 3, about 4, about 5, or more seconds. Preferably, the puff sound is about 3 seconds. The memory may store any number of puff sounds according to the space available in the memory. In some embodiments, the memory stores 1, 2, 3, 4, 5, or more puff sounds. Preferably, the memory stores 4 puff sounds. In some embodiments, each puff sound may be stored as about 48 kilobytes of compressed sound data. In some embodiments, a plurality of puff sounds may be stored as about 192 kilobytes of compressed sound data.

Additionally, or alternatively, the memory may have enough space to store information for the user. Information for the user may be instructions for using or maintaining the aerosol-generating device. Each of the instructions may have a duration ranging between about 1 second and about 120 seconds. The memory may store one or more instructions according to the space available in the memory. Preferably, an instruction has a duration of about 1 minute.

The memory may be small enough to fit into a body of the aerosol-generating substrate while storing one or more sounds. The memory may have sufficient space to store at least about 4 different puff sounds and about 1 minute of information. The memory may fit into an area of less than or equal to about 5 mm x about 5 mm. Preferably, the memory fits into a volume of less than about 3 mm x about 2 mm.

The substrate sensor may be disposed adjacent to the cavity. The substrate sensor may be utilized by the controller to automatically detect a type of aerosol-generating substrate received in the cavity.

The aerosol-generating device may operate in one or more modes. Non-limiting examples of modes may include a puff sound mode, an information mode, a substrate detection mode, a data download mode, and a configuration mode.

Each of the modes may be enabled or disabled individually or in combination with one or more other modes. A user action may be used to enable or disable one or more of the different modes or to initiate functions in the one or more modes. For example, the user may engage the actuator in various manners to select various modes or to initiate various functions.

A user action may be an action detectable by the controller. In some embodiments, a user action may include one or more puffs by the user. In some embodiments, the user action may include coupling the aerosol-generating substrate to the aerosolizer. In some embodiments, the user action may include engaging the actuator.

The same or different user actions may be used to initiate various functionality of each mode. For example, in a puff sound mode, sound may be initiated in response to a user puff, whereas in an information mode, sound may be initiated by an engagement of the actuator.

One of the puff sound modes may be a specific puff sound mode. The controller may detect one or more user puffs. In response to detecting the one or more puffs, sound data representing the specific puff sound may be read from the memory. The sound generator may be initiated by the controller into producing the specific puff sound based on the sound data.

One of the puff sound modes may be a generic puff sound mode. The generic sound produced may be white noise or other noise to mask other sounds. In response to detecting one or more puffs, sound data representing the generic sound may be read from the memory. The sound generator may be initiated by the controller into producing the sound emulating the generic sound, such as white noise, based on the sound data.

One of the information modes may be an instruction mode. The controller may read sound data representing an instruction from the memory. The sound generator may be initiated by the controller into producing the sound based on the sound data. In some embodiments, in response to the aerosol-generating device being powered on, the controller may initiate production of sound representing the instruction. In some embodiments, in response to one or more user engagements of the actuator (for example, four consecutive presses), the controller may initiate production of sound representing the instruction.

One of the information modes may be an error message mode to provide an error status of the aerosol-generating device. The controller may supervise a user action and detect an error affecting the aerosol-generating device. In response to detecting the error, the controller may read sound data indicating an error from the memory. The sound generator may be initiated by the controller into producing the sound representing an error message based on the sound data. The error message may be provided as a voice message.

One of the information modes may be an operational message mode to provide the user with the aerosol-generating device status. A user message may be provided as one or more beeps, one or more tones, or preferably, a voice message. The controller may detect a user action. In response to the user action, the controller may read sound data associated with the user action from the memory. In some embodiments, in response to a quick engagement of the actuator, the controller may initiate production of a voice message indicating a battery test (for example, "Button pressed, starting battery test, device will not start"), whereas a more prolonged engagement of the actuator may initiate aerosolization and a different voice message (for example, "The device is starting, you can start using it in 30 seconds"). In some embodiments, device status messages to the user may include an indication of puffs left, an indication of a user interaction with the device, and an indication of a function of the device.

One of the modes may be a substrate detection mode. The controller may determine a type of aerosol-generating substrate automatically or after a user action. In some embodiments, in response to the aerosol-generating substrate being coupled to the aerosolizer, the controller determines the type of substrate automatically using the substrate sensor. In response to detecting one or more puffs, the controller may select the specific puffing sound stored in memory that matches, or is associated with, the type of substrate detected.

One of the modes may be a substrate selection mode. The controller may receive information representing the type of aerosol-generating substrate. The substrate type information may be received using the communication interface and may be provided by the user interface device.

One of the modes may be a data download mode. The controller may use the communication interface to receive data, such as sound data to be stored in the memory, which may be provided by the user interface device.

One of the modes may be a configuration mode. The controller may receive a configuration using the communication interface from, for example, a user interface device. In some embodiments, the configuration may define one or more of a type of aerosol-generating substrate, a specific puff sound mode, a generic puff sound mode, an instruction mode, an error message mode, a status message mode, a substrate detection mode, a substrate selection mode, a data download mode, a configuration mode, a volume level, and an audio quality level (for example, based on bitrate and compression level). The configuration mode may be activated, for example, by a pattern of engagement of the actuator or other suitable user input detectable by the controller.

The sound generated that emulates a conventional use of a smoking material or white noise may be modulated based on a user's puff. The user's puff may be characterized by a puff profile. The puff profile may be determined based on one or more measured puffs from the user using the puff sensor. The rate or duration of the puff or puffs may be represented by the puff profile. The puff profile may be used to change a characteristic of the sound, such as frequency or intensity. For example, a higher puff rate, or higher inhalation rate, may be used to increase intensity (for example, the volume) of the sound generated. The change in the sound may emulate the change in conventional uses of smoking material.

The puff sensor may be integral with the aerosolizer. The aerosolizer may have a characteristic measurable by the controller that indicates a characteristic of the user's puff. The measurable characteristic of the aerosolizer may be used to determine the puff profile. In some embodiments, the aerosolizer (for example, in the form of a heat blade) may change a resistance characteristic when the user puffs due to a temperature drop of the aerosol-generating substrate and the aerosolizer. The higher rate of puff may decrease the temperature and the resistance of the aerosolizer. The decreased resistance may result in a higher current flowing through the aerosolizer. The current characteristic may be measured to represent a puff profile.

The communication interface may be used to communicate to a user interface device. Any suitable communication interface may be used to communicate to the user interface device. The communication interface may be wireless, wired, or both. An example of a wireless interface utilizes Bluetooth, such as Bluetooth Low Energy (BLE). An example of a wired interface utilizes a universal serial bus (USB). In some embodiments, the communication interface may be disposed on the same silicon chip or integrated circuit as the processor.

The aerosol-generating device may be used without the user interface device. However, the user interface device may enable various functionalities and provide a user-friendly manner of configuring the aerosol-generating device.

The user interface device may be any suitable device to accept a user selection and communicate with the aerosol-generating device. The user interface device may communicate a configuration to the aerosol-generating device. In some embodiments, the user interface device may be a smartphone or tablet with an application to facilitate communication with the aerosol-generating device.

The user interface device may include a communication interface, a display, and a controller. The communication interface may communicate with the communication interface of the aerosol-generating device. The display may include a user interface engageable by a user to configure the aerosol-generating device, such as a touch screen, to accept user selections. The display may present one or more graphical elements to configure the aerosol-generating device. The controller may be operably coupled to the display and the communication interface.

The controller may display the one or more graphical elements on the display. The controller may receive the user selection using the one or more graphical elements to configure the aerosol-generating device. The controller may communicate with the aerosol-generating device using the communication interface to configure the aerosol-generating device based on the user selection.

A method of using the MEMS sound generator for an aerosol-generating device may include providing the aerosol-generating device with the MEMS sound generator. The method may further include detecting an action of a user of the aerosol-generating device. The method may also include producing the sound using the MEMS sound generator in response to the action of the user.

A non-transitory computer readable storage medium may include a computer program stored which, when run on programmable electric circuitry, causes the programmable electric circuitry to detect an action of a user of an aerosol-generating device and initiate production of a sound using a MEMS sound generator in response to the action of the user.

A system may include one or more of the aerosol-generating devices and user interface devices described herein. However, the aerosol-generating device may be used without the user interface device.

The schematic drawings are not necessarily to scale and are presented for purposes of illustration and not limitation. Referring now to the drawings, in which some aspects of the present invention are illustrated.

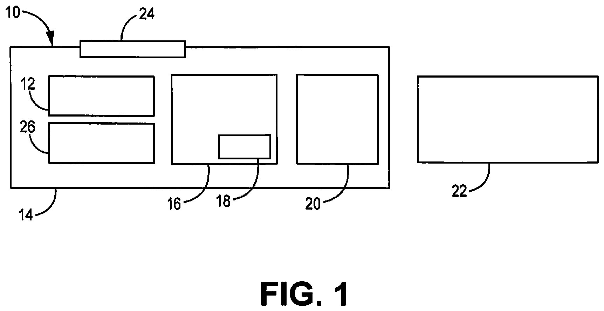

**FIG. 1** is a diagram of a device **10** including a MEMS sound generator **12.** The device **10** may be described as an aerosol-generating device. The sound generator **12** may be disposed within a housing **14** of the device **10.** The device **10** may include a controller **16.** The device **10** may include a memory **18,** which may be considered part of the controller **16.** The controller **16** may initiate the sound generator **12** into producing sound, such as high-fidelity sound. The controller **16** may initiate the aerosolizer **20** into aerosolizing an aerosol-generating substrate **22** coupled to the aerosolizer. The initiation of the sound and the aerosolizing may be associated. For example, sound may be generated by the sound generator **12** while the aerosol is generated in a concurrent, or at least overlapping, manner. As shown, the aerosol-generating substrate **22** may be removably coupled to the aerosolizer **20.** The aerosol-generating substrate **22** may be at least partially received in the housing **14** of the device **10.**

The device **10** may include an actuator **24.** The actuator **24** may be a button switch. The actuator **24** may be engaged (for example, a button switch may be depressed by a user's finger) to initiate one or more functions of the device **10.** For example, the actuator **24** may initiate one or both of sound production and aerosolizing. The sound production may include information for a user of the device **10.**

The device **10** may include a puff sensor **26.** The puff sensor **26** may detect a user puff on the device **10.** Upon detecting a puff, the controller **16** may initiate one or both of sound production and aerosolizing. The sound production may include a puff sound, which may be specific to the substrate **22** or generic (for example, white noise).

A power source (not shown) may be included in the device **10.** However, external power sources are also contemplated. The power source may provide power to the aerosolizer **20** for the process of aerosolization.

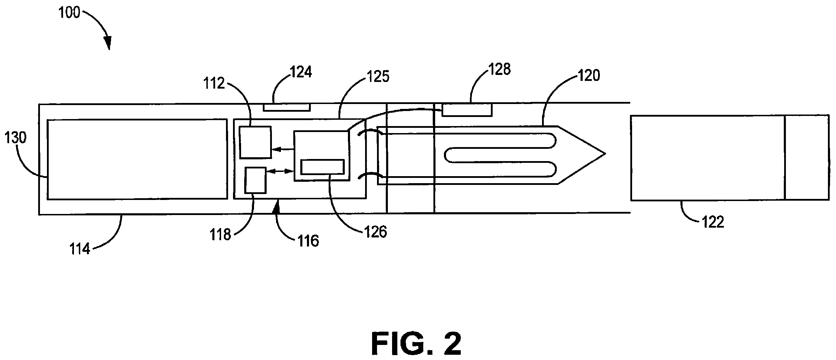

**FIG. 2** is a diagram of an aerosol-generating device **100** including a MEMS sound generator **112** used with an aerosol-generating substrate **122** in the form of a heat stick. The heat stick may contain a smoking material that may be heated but not burned to generate aerosol. The device **100** includes an aerosolizer **120** in the form of a heating blade to aerosolize the substrate **122.** As shown, the substrate **122** may be removably coupled to the aerosolizer **120.**

The device **100** may include a housing **114,** a controller **116,** a memory **118** to store one or more sounds, an actuator **124,** a processor **125,** a communication interface **126,** a substrate sensor **128,** and a power source **130.** The communication interface **126** may be disposed on the same silicon chip or integrated circuit as the processor **125.** The power source **130** may be a battery.

The substrate sensor **128** may be operatively coupled to the controller **116 to** provide an indication of the type of substrate **122** that may be coupled to the aerosolizer **120.** The sensor **128** may read an identifier, such as a barcode or color, coupled to the substrate **122.**

The communication interface **126** may receive an indication of the type of substrate **122** that may be coupled to the aerosolizer **120.**

In addition to aerosolizing, the aerosolizer **120** may be used to measure one or more characteristics of a user's puff, which may be described as a puff profile. For example, the processor **125** of the controller **116** may be used to measure a resistance change in the aerosolizer **120,** which may correspond to an inhalation rate as the user puffs. The processor **125** of the controller **116** may be used to modulate the sound generated by the sound generator **112,** for example in frequency content or intensity, based on the puff profile. Additionally, or alternatively, an independent puff sensor (not shown) may be included in the device **100.**

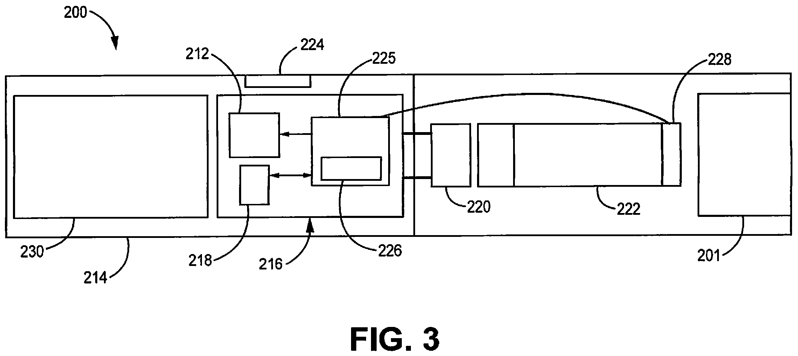

**FIG. 3** is a diagram of an aerosol-generating device **200** including a MEMS sound generator **212** used with an aerosol-generating substrate **222** in the form of a liquid substrate (for example, contained in a cartridge). The substrate **222** may evaporate upon heating to generate aerosol. The device **100** includes an aerosolizer **220** in the form of a heater adjacent to the substrate **222.** As shown, the cartridge containing the substrate **222** may be removably coupled to the aerosolizer **220.**

The device **200** may include a mouthpiece **201,** a housing **214,** a controller **216,** a memory **218** to store one or more sounds, an actuator **224,** a processor **225,** a communication interface **126,** and a power source **230.** The communication interface **226** may be disposed on the same silicon chip or integrated circuit as the processor **225.** The power source **230** may be a battery.

The controller **216** may communicate with a substrate identifier **228** coupled to the substrate **222** (for example, coupled to the cartridge). For example, the substrate identifier **228** may be a wireless tag (for example, an RFID tag). The controller **216** may receive, or read, an identifier from the substrate identifier **228** to provide an indication of the type of substrate **222** that may be coupled to the aerosolizer **220.** The communication interface **226** may receive an indication of the type of substrate **222** that may be coupled to the aerosolizer **220,** for example, from the substrate identifier **228** or a user interface device.

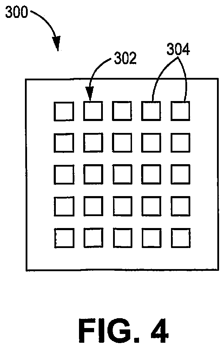

**FIG. 4** is a diagram of a MEMS sound generator **300.** The sound generator **300** may include an array **302** of pressure-generating drivers **304.** Each driver **304** may generate one or more frequencies. Each driver **304** in the array **302** may produce a different frequency. The sound generator **300** may receive a digital signal or data as input. The sound generator **300** may utilize digital sound reconstruction (DSR) to provide high-fidelity sound. As illustrated, the array **302** may be square shaped and have an equal number of rows and columns. However, any suitable shape and number of rows and columns may be used to provide the high-fidelity sound.

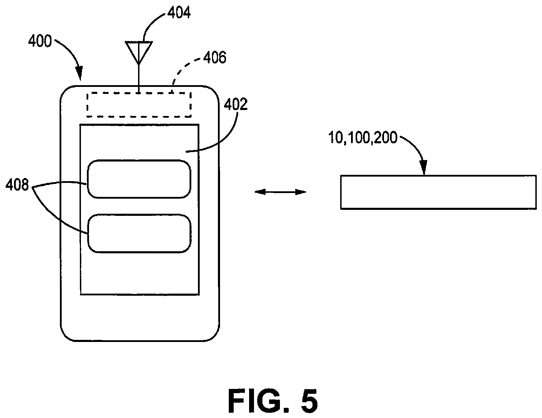

**FIG. 5** is a diagram of a user interface device **400** in communication with an aerosol-generating device **10, 100, 200.** The device **400** may include a display **402** with a user interface to display one or more graphical elements **408.** The user interface may include a touch screen. The device **400** may include a communication interface **404** operatively coupled to a controller **406** to communicate with the aerosol-generating device **10, 100, 200.** As illustrated, the communication interface **404** may be wireless. However, a wired interface is also contemplated. The controller **406** may communicate with the aerosol-generating device **10, 100, 200** using the communication interface **404** to, for example, provide a configuration or sound data to the aerosol-generating device.

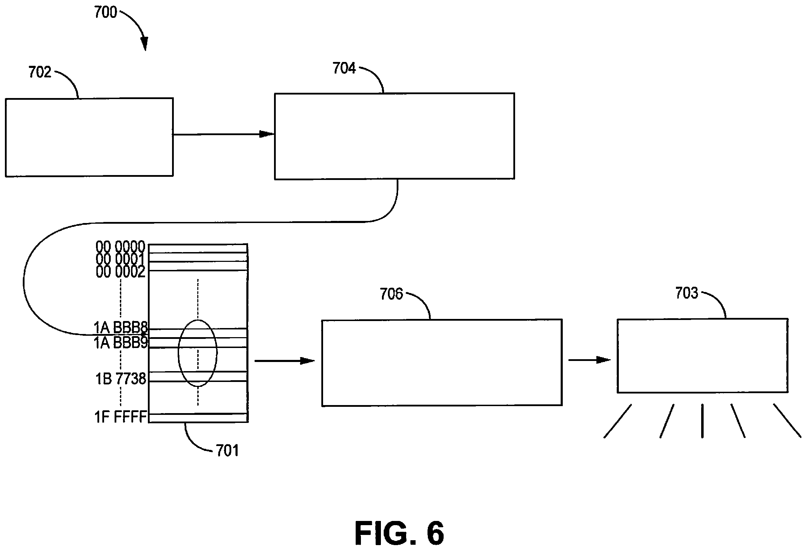

**FIG. 6** is a flowchart of a method **700** to detect a puff and produce a puff sound. In process **702,** the puff may be detected, which may be carried out by hardware (for example, a puff sensor or heating blade) and a controller subroutine. The controller may include a microcontroller. In process **704,** once a puff is detected, a puff sound address may be identified and the puff sound data at the puff sound address may be retrieved from memory **701,** which may be carried out in a controller subroutine. In process **706,** the audio data may be sent to the sound generator **703** to produce the puff sound. The sound generator may be a microspeaker.

The specific embodiments described above are intended to illustrate the invention. It is to be understood that the specific embodiments described above are not intended to be limiting.

All scientific and technical terms used herein have meanings commonly used in the art unless otherwise specified. The definitions provided herein are to facilitate understanding of certain terms used frequently herein.

The term "coupled" refers to elements being attached to each other either directly (in direct contact with each other) or indirectly (having one or more elements between and attaching the two elements).

The term "operatively coupled" refers to elements being associated in a manner capable of performing a function involving the elements.

As used herein, the singular forms "a," "an," and "the" encompass embodiments having plural referents, unless the content clearly dictates otherwise.

As used herein, "or" is generally employed in its sense including "and" unless the content clearly dictates otherwise. The term "or" means one or all the listed elements or a combination of any two or more of the listed elements.

As used herein, "have," "having," "include," "including," "comprise," "comprising" or the like are used in their open-ended sense, and generally mean "including, but not limited to". It will be understood that "consisting essentially of," "consisting of," and the like are subsumed in "comprising," and the like.

The words "preferred" and "preferably" refer to embodiments of the invention that may afford certain benefits, under certain circumstances. However, other embodiments may also be preferred, under the same or other circumstances. Furthermore, the recitation of one or more preferred embodiments does not imply that other embodiments are not useful, and is not intended to exclude other embodiments from the scope of the claims.

## Claims

1. An aerosol-generating device (10) comprising:
an aerosolizer (20) to generate aerosol from an aerosol-generating substrate (22);
a MEMS sound generator (12) comprising an array (302) of pressure-generating drivers (304) configured to use digital sound reconstruction (DSR) to use a summation of discrete pulses to produce sound; and
a controller (16) operatively coupled to initiate the MEMS sound generator (12) into producing sound.

2. The device (10) according to claim 1, wherein the MEMS sound generator (12) compresses air to produce the sound.

3. The device (10) according to claim 1 or 2, wherein the array (302) of pressure-generating drivers (304) produces a plurality of different frequencies.

4. The device (10) according to any preceding claim, further comprising an actuator (24) operatively coupled to the controller (16) and wherein the MEMS sound generator (12) produces sound information in response to engagement of the actuator (24).

5. The device (10) according to any preceding claim, further comprising a puff sensor (26) operatively coupled to the controller (16) to measure a user puff.

6. The device (10) according to claim 5, wherein the controller (16) is further configured to measure a characteristic of the aerosolizer (20) to measure the user puff.

7. The device (10) according to claim 5 or claim 6, wherein the controller (16) is configured to change a characteristic of the sound based on a puff rate or an inhalation rate determined from measured user puffs.

8. The device (10) according to any preceding claim, further comprising the aerosol-generating substrate (22), the substrate comprising a nicotine material.

9. A method for use with an aerosol-generating device (10), the method comprising:
detecting an action of a user using the aerosol-generating device (10); and
producing sound using a MEMS sound generator (12) in response to the action of the user, wherein the MEMS sound generator (12) comprises an array (302) of pressure-generating drivers (304) configured to use digital sound reconstruction (DSR) to use a summation of discrete pulses to produce sound.

10. The method according to claim 9, wherein producing sound comprises producing sound that comprises information for a user, preferably information in the produced sound comprises a voice message.

11. The method according to claim 10, wherein information in the produced sound provides an instruction to use or maintain the aerosol-generating device (10), a status of the aerosol-generating device (10), or both.

12. A non-transitory computer readable storage medium including a computer program stored which, when run on programmable electric circuitry of an aerosol-generating device (10), causes the programmable electric circuitry to execute a method comprising:
detecting an action of a user using the aerosol-generating device (10); and
producing sound using a MEMS sound generator (12) in response to the action of the user, wherein the MEMS sound generator (12) comprises an array (302) of pressure-generating drivers (304) configured to use digital sound reconstruction (DSR) to use a summation of discrete pulses to produce sound.

13. A system comprising:
the aerosol-generating device (10) according to any one of claims 1 to 8; and
a user interface device (400) comprising:
a communication interface (404) to communicate with the aerosol-generating device (10);
a display (402) comprising a user interface to present one or more graphical elements (408) to configure the aerosol-generating device (10); and
a controller (406) f+êt operably coupled to the display (402) and communication interface (404), the controller (406) configured to:
display the one or more graphical elements on the display (402);
allow a user selection using the one or more graphical elements via the user interface (400) to configure the aerosol-generating device (10); and
communicate with the aerosol-generating device (10) using the communication interface (404) to configure the aerosol-generating device (10) based on the user selection.

14. The system according to claim 13, wherein the controller (406) of the user interface (400) device is configured to communicate a configuration to the aerosol-generating device (10) defining one or more of: a type of aerosol-generating substrate (22), a specific puff sound mode, a generic puff sound mode, an instruction mode, an error message mode, an operational message mode, a substrate detection mode, a substrate selection mode, a data download mode, a configuration mode, a volume level, and an audio quality level.

15. The method of claim 9 or the non-transitory computer readable storage medium of claim 12, wherein producing the sound using the MEMS sound generator (12) comprises changing a characteristic of the sound based on a puff rate or an inhalation rate determined from measured user puffs.

## Patentansprüche

1. Aerosolerzeugungsvorrichtung (10), aufweisend:
einen Aerosolerzeuger (20) zum Erzeugen von Aerosol aus einem aerosolerzeugenden Substrat (22);
einen MEMS-Tonerzeuger (12), umfassend eine Anordnung (302) von druckerzeugenden Treibern (304), die zum Verwenden der digitalen Tonrekonstruktion (DTR) ausgelegt ist, um eine Summierung diskreter Impulse zur Tonerzeugung zu verwenden; und
eine wirkgekoppelte Steuerung (16) zum Initiieren des MEMS-Tonerzeugers (12) zur Tonerzeugung.

2. Vorrichtung (10) nach Anspruch 1, wobei der MEMS-Tonerzeuger (12) zum Erzeugen des Tons Luft verdichtet.

3. Vorrichtung (10) nach Anspruch 1 oder 2, wobei die Anordnung (302) der druckerzeugenden Treiber (304) eine Vielzahl von unterschiedlichen Frequenzen erzeugt.

4. Vorrichtung (10) nach einem beliebigen vorhergehenden Anspruch, ferner umfassend einen mit der Steuerung (16) wirkgekoppelten Aktuator (24), wobei der MEMS-Tonerzeuger (12) in Reaktion auf den Eingriff des Aktuators (24) Toninformationen erzeugt.

5. Vorrichtung (10) nach einem beliebigen vorhergehenden Anspruch, ferner umfassend einen mit der Steuerung (16) wirkgekoppelten Zugsensor (26) zum Messen eines Benutzerzuges.

6. Vorrichtung (10) nach Anspruch 5, wobei die Steuerung (16) ferner zum Messen einer Eigenschaft des Aerosolerzeugers (20) zum Messen des Benutzerzuges ausgelegt ist.

7. Vorrichtung (10) nach Anspruch 5 oder Anspruch 6, wobei die Steuerung (16) zum Ändern einer Eigenschaft des Tons basierend auf einer Zuggeschwindigkeit oder einer aus gemessenen Benutzerzügen ermittelten Inhalationsgeschwindigkeit ausgelegt ist.

8. Vorrichtung (10) nach einem beliebigen vorhergehenden Anspruch, ferner umfassend das aerosolerzeugende Substrat (22), wobei das Substrat ein Nikotinmaterial umfasst.

9. Verfahren zum Gebrauch mit einer Aerosolerzeugungsvorrichtung (10), das Verfahren umfassend:
Detektieren einer Aktion eines Benutzers, der die Aerosolerzeugungsvorrichtung (10) verwendet; und
Erzeugen von Ton unter Verwendung eines MEMS-Tonerzeugers (12) in Reaktion auf die Aktion des Benutzers, wobei der MEMS-Tonerzeuger (12) eine Anordnung (302) von druckerzeugenden Treibern (304) aufweist, die zum Verwenden von digitaler Tonrekonstruktion (DTR) ausgelegt ist, um eine Summierung von diskreten Impulsen zur Tonerzeugung zu verwenden.

10. Verfahren nach Anspruch 9, wobei das Erzeugen von Ton das Erzeugen von Ton umfasst, der Informationen für einen Benutzer aufweist, wobei die Informationen in dem erzeugten Ton bevorzugt eine Sprachnachricht umfassen.

11. Verfahren nach Anspruch 10, wobei die Informationen in dem erzeugten Ton eine Anweisung zum Gebrauch oder zur Wartung der Aerosolerzeugungsvorrichtung (10), einen Status der Aerosolerzeugungsvorrichtung (10) oder beides vorsieht.

12. Nicht-flüchtiges, computerlesbares Speichermedium, beinhaltend ein gespeichertes Computerprogramm, das bei Ausführung in einem programmierbaren elektrischen Schaltkreis einer Aerosolerzeugungsvorrichtung (10) den programmierbaren elektrischen Schaltkreis zum Ausführen eines Verfahrens veranlasst, umfassend:
Detektieren einer Aktion eines Benutzers, der die Aerosolerzeugungsvorrichtung (10) verwendet; und
Erzeugen von Ton unter Verwendung eines MEMS-Tonerzeugers (12) in Reaktion auf die Aktion des Benutzers, wobei der MEMS-Tonerzeuger (12) eine Anordnung (302) von druckerzeugenden Treibern (304) aufweist, die zum Verwenden von digitaler Tonrekonstruktion (DTR) ausgelegt ist, um eine Summierung von diskreten Impulsen zur Tonerzeugung zu verwenden.

13. System, umfassend:
die Aerosolerzeugungsvorrichtung (10) nach einem der Ansprüche 1 bis 8; und
ein Benutzerschnittstellenvorrichtung (400), umfassend:
eine Kommunikationsschnittstelle (404) zum Kommunizieren mit der Aerosolerzeugungsvorrichtung (10);
eine Anzeige (402), umfassend eine Benutzeroberfläche zum Darstellen eines oder mehrerer grafischer Elemente (408) zum Konfigurieren der Aerosolerzeugungsvorrichtung (10); und
eine mit der Anzeige (402) und der Kommunikationsschnittstelle (404) wirkgekoppelte Steuerung (406), wobei die Steuerung (406) ausgelegt ist zum:
Anzeigen des einen oder der mehreren grafischen Elemente auf der Anzeige (402);
Ermöglichen einer Benutzerauswahl unter Verwendung des einen oder der mehreren grafischen Elemente über die Benutzeroberfläche (400) zum Konfigurieren der Aerosolerzeugungsvorrichtung (10); und
Kommunizieren mit der Aerosolerzeugungsvorrichtung (10) unter Verwendung der Kommunikationsschnittstelle (404) zum Konfigurieren der Aerosolerzeugungsvorrichtung (10) basierend auf der Benutzerauswahl.

14. System nach Anspruch 13, wobei die Steuerung (406) der Benutzeroberflächenvorrichtung (400) zum Kommunizieren einer Konfiguration an die Aerosolerzeugungsvorrichtung (10) ausgelegt ist, die eines oder mehrere definiert von: einer Art von aerosolerzeugendem Substrat (22), einem spezifischen Zuggeräuschmodus, einem generischen Zuggeräuschmodus, einem Anweisungsmodus, einem Fehlermeldungsmodus, einem Betriebsmeldungsmodus, einem Substratdetektionsmodus, einem Substratauswahlmodus, einem Datendownloadmodus, einem Konfigurationsmodus, einem Lautstärkepegel und einem Audioqualitätspegel.

15. Verfahren nach Anspruch 9 oder nicht-flüchtiges computerlesbares Speichermedium nach Anspruch 12, wobei das Erzeugen des Tons unter Verwendung des MEMS-Tonerzeugers (12) das Ändern einer Eigenschaft des Tons basierend auf einer Zuggeschwindigkeit oder einer aus gemessenen Benutzerzügen ermittelten Inhalationsgeschwindigkeit umfasst.

## Revendications

1. Dispositif de génération d'aérosol (10) comprenant :
un générateur d'aérosol (20) destiné à générer un aérosol à partir d'un substrat de génération d'aérosol (22) ;
un générateur de son MEMS (12) comprenant un réseau (302) de circuits d'attaque de génération de pression (304) configuré pour utiliser une reconstruction de son numérique (DSR) pour utiliser une somme d'impulsions discrètes pour produire du son ; et
un dispositif de commande (16) couplé de manière fonctionnelle pour amorcer le générateur de son MEMS (12) dans la production de son.

2. Dispositif (10) selon la revendication 1, dans lequel le générateur de son MEMS (12) comprime l'air pour produire le son.

3. Dispositif (10) selon la revendication 1 ou 2, dans lequel le réseau (302) de dispositifs d'attaque de génération de pression (304) produit une pluralité de fréquences différentes.

4. Dispositif (10) selon l'une quelconque des revendications précédentes, comprenant en outre un actionneur (24) couplé de manière fonctionnelle au dispositif de commande (16) et dans lequel le générateur de son MEMS (12) produit des informations sonores en réponse à l'enclenchement de l'actionneur (24).

5. Dispositif (10) selon l'une quelconque des revendications précédentes, comprenant en outre un capteur de bouffée (26) couplé de manière fonctionnelle au dispositif de commande (16) pour mesurer une bouffée d'utilisateur.

6. Dispositif (10) selon la revendication 5, dans lequel le dispositif de commande (16) est en outre configuré pour mesurer une caractéristique du générateur d'aérosol (20) pour mesurer la bouffée de l'utilisateur.

7. Dispositif (10) selon la revendication 5 ou la revendication 6, dans lequel le dispositif de commande (16) est configuré pour changer une caractéristique du son sur la base d'un taux de bouffées ou d'un taux d'inhalation déterminé à partir de bouffées d'utilisateur mesurées.

8. Dispositif (10) selon l'une quelconque des revendications précédentes, comprenant en outre le substrat de génération d'aérosol (22), le substrat comprenant une matière de nicotine.

9. Procédé destiné à être utilisé avec un dispositif de génération d'aérosol (10), le procédé comprenant :
la détection d'une action d'un utilisateur à l'aide du dispositif de génération d'aérosol (10) ; et
la production d'un son à l'aide d'un générateur de son MEMS (12) en réponse à l'action de l'utilisateur, dans lequel le générateur de son MEMS (12) comprend un réseau (302) de circuits d'attaque de génération de pression (304) configuré pour utiliser une reconstruction de son numérique (DSR) pour utiliser une somme d'impulsions discrètes pour produire un son.

10. Procédé selon la revendication 9, dans lequel la production d'un son comprend la production d'un son qui comprend des informations pour un utilisateur, de préférence des informations dans le son produit comprennent un message vocal.

11. Procédé selon la revendication 10, dans lequel les informations dans le son produit fournit une instruction pour utiliser ou entretenir le dispositif de génération d'aérosol (10), un état du dispositif de génération d'aérosol (10), ou les deux.

12. support de stockage lisible par ordinateur non transitoire comprenant un programme informatique stocké qui, lorsqu'il est exécuté sur un circuit électrique programmable d'un dispositif de génération d'aérosol (10), amène le circuit électrique programmable à exécuter un procédé comprenant :
la détection d'une action d'un utilisateur à l'aide du dispositif de génération d'aérosol (10) ; et
la production d'un son à l'aide d'un générateur de son MEMS (12) en réponse à l'action de l'utilisateur, dans lequel le générateur de son MEMS (12) comprend un réseau (302) de circuits d'attaque de génération de pression (304) configuré pour utiliser une reconstruction de son numérique (DSR) pour utiliser une somme d'impulsions discrètes pour produire un son.

13. Système comprenant :
un dispositif de génération d'aérosol (10) selon l'une quelconque des revendications 1 à 8 ; et
un dispositif d'interface utilisateur (400) comprenant :
une interface de communication (404) pour communiquer avec le dispositif de génération d'aérosol (10) ;
un affichage (402) comprenant une interface utilisateur pour présenter un ou plusieurs éléments graphiques (408) pour configurer le dispositif de génération d'aérosol (10) ; et
un dispositif de commande (406) couplé de manière fonctionnelle à l'affichage (402) et à l'interface de communication (404), le dispositif de commande (406) étant configuré pour :
l'affichage des un ou plusieurs éléments graphiques sur l'affichage (402) ;
permettre à une sélection utilisateur à l'aide des un ou plusieurs éléments graphiques via l'interface utilisateur (400) afin de configurer le dispositif de génération d'aérosol (10) ; et
communiquer avec le dispositif de génération d'aérosol (10) à l'aide de l'interface de communication (404) afin de configurer le dispositif de génération d'aérosol (10) sur la base de la sélection utilisateur.

14. Système selon la revendication 13, dans lequel le dispositif de commande (406) du dispositif d'interface utilisateur (400) est configuré pour communiquer une configuration au dispositif de génération d'aérosol (10) définissant un ou plusieurs parmi : un type de substrat de génération d'aérosol (22), un mode de son de bouffée spécifique, un mode de son de bouffée générique, un mode d'instruction, un mode de message d'erreur, un mode de message fonctionnel, un mode de détection de substrat, un mode de sélection de substrat, un mode de téléchargement de données, un mode de configuration, un niveau de volume et un niveau de qualité audio.

15. Procédé selon la revendication 9 ou support de stockage lisible par ordinateur non transitoire selon la revendication 12, dans lequel la production du son à l'aide du générateur de son MEMS (12) comprend le changement d'une caractéristique du son sur la base d'un taux de bouffées ou d'un taux d'inhalation déterminé à partir de bouffées d'utilisateur mesurées.
